# DEMANDE DE BREVET EUROPEEN

(11) **EP 1 095 622 A1**
(43) Date de publication de la demande: **02.05.2001**
(21) Numéro de dépôt: 99810983.9
(22) Date de dépôt: 29.10.1999
(51) Int. Cl.: A61B 17/04

(54) **Instrument endoscopique de suture**

(71) Demandeur: Biomedix S.A., 1701 Fribourg (CH)
(72) Inventeur: Godin, Norman, Dr., 1208 Geneve (CH)
(74) Mandataire: Savoye, Jean-Paul

(57) **Abrégé**

Cet instrument endoscopique de suture comprend un corps creux allongé (1) dont l'extrémité distale (3) comporte une aiguille de suture courbe (5) et des moyens de guidage (4) pour guider ladite aiguille (5) selon une trajectoire courbe s'étendant latéralement hors dudit corps creux allongé (1) et dont l'extrémité proximale (6) comporte un organe d'actionnement (8) pour déplacer ladite aiguille (5) par rapport aux dits moyens de guidage (4), un organe de transmission (9) reliant ledit organe d'actionnement (8) à ladite aiguille de suture (5) à travers un canal longitudinal (2) traversant ledit corps creux (1). Ledit corps creux (1) comporte un second canal longitudinal (10) dont une première extrémité s'arrête à proximité de ladite extrémité distale (3) et occupe une position latérale à l'axe longitudinal dudit corps creux (1), pour permettre le passage d'un endoscope.

## Description

La présente invention se rapporte à un instrument endoscopique de suture, comprenant un corps creux allongé dont une extrémité distale comporte une aiguille de suture et dont l'extrémité proximale comporte des moyens d'actionnement pour commander ladite aiguille de suture des moyens de transmission reliant ledit organe d'actionnement à ladite extrémité distale à travers un canal longitudinal traversant ledit corps creux.

Un tel instrument est décrit dans différents brevets, notamment dans le EP-0 674 875. Il permet de réaliser des sutures sur des tissus situés à l'intérieur d'un organisme vivant en disposant l'extrémité distale de l'instrument contre le tissu à suturer. En sortant latéralement du corps allongé de l'instrument, l'aiguille courbe de suture peut traverser le tissu de l'organe dans lequel la suture doit être réalisée. Ensuite on peut ressortir le fil de suture du corps creux de l'instrument pour faire un noeud que l'on pousse avec l'extrémité de l'instrument puis que l'on serre. Il s'agit là d'une technique de nouage connue dans les interventions en chirurgie laparoscopique.

Pour pouvoir travailler avec un tel instrument, il est nécessaire d'amener séparément un endoscope rigide à travers la paroi abdominale à proximité de l'extrémité distale de l'instrument de suture pour transmettre une image de la zone de suture. Avec cet instrument, il faut avoir accès à la zone de suture par deux chemins différents, impliquant deux petits orifices de laparotomie. Cette technique ne peut donc pas être utilisée dans le cas où l'intervention n'est réalisable que par des conduits naturels de l'organisme vivant.

On a par ailleurs déjà proposé d'associer un dispositif d'agrafage à l'extrémité d'un endoscope en vue de poser des agrafes dans du tissu gastro-intestinal, comme décrit en particulier par C. Paul Swain et al. en pages 338-339 volume 35, No. 4, 1989 de Gastrointestinal Endoscopy. A cet effet, le dispositif d'agrafage comporte une fente latérale reliée à une source d'aspiration. Cette fente est appliquée contre le tissu gastro-intestinal, de sorte qu'un pli de ce tissu est aspiré dans la fente et l'agrafe est fixée à travers ce pli.

Un appareil de suture basé sur le même principe et provenant des mêmes auteurs est décrit en pages 36-38, volume 32, No. 1, 1986 de Gastrointestinal Endoscopy.

Ce type d'appareil n'est malheureusement pas adapté à la pose d'une prothèse pour s'opposer au reflux gastrique dans l'oesophage par exemple, telle que celle décrite dans le WO 96/29954. Il n'est pas possible d'aspirer simultanément dans la fente le tissu dans lequel la prothèse doit être fixée et le bord de cette prothèse. En effet, cette dernière étant réalisée en un élastomère imperméable à l'air, elle serait aspirée dans la fente de l'instrument, mais elle ne permettrait pas à la source d'aspiration d'aspirer le tissu adjacent à la prothèse en raison de l'imperméabilité de l'élastomère dans lequel elle est réalisée. L'appareil de suture par aspiration est également mal adapté quand on veut rapprocher deux bords d'une plaie et faire un point de suture, car en aspirant la muqueuse, on tend à élargir la plaie qu'on désire coudre.

Le but de la présente invention est d'apporter une solution permettant d'effectuer une suture endoscopique uniquement par les voies naturelles d'un organisme vivant, humain ou animal, qui ne présente cependant pas les limitations des dispositifs susmentionnés, notamment en ce qui concerne la possibilité de fixer une prothèse à la paroi de la base de l'oesophage, pour s'opposer au reflux gastrique dans celui-ci. Elle a également pour but de permettre de suturer les ulcères gastriques ou des plaies chirurgicales après excision de tumeurs bénignes importantes comme de gros polypes coliques.

A cet effet, la présente invention a pour objet un instrument endoscopique de suture selon la revendication 1.

L'avantage de cet instrument est de ne plus nécessiter de pratiquer des ouvertures à travers le corps du patient pour effectuer la suture. Un autre avantage est celui de rendre possible la couture d'un corps étranger, notamment un corps en un matériau imperméable à l'air contre le tissu vivant, ce que ne permettent pas de faire les instruments connus utilisant l'aspiration du tissu dans une cavité. Ceci présente une grande importance dans certains cas, comme par exemple pour la fixation d'une prothèse du type susmentionné au bas de l'oesophage en vue d'empêcher le reflux gastrique. En effet, une telle intervention ne peut être faite qu'en passant par l'oesophage et il ne serait pas acceptable de devoir recourir à une laparotomie pour la réaliser, d'où l'importance de la solution proposée. Un autre avantage de cet instrument est de permettre de suturer des ulcères ou des plaies consécutives à une excision endoscopique de lésion avec un courant électrique coupant et cautérisant par exemple.

Le dessin annexé illustre, schématiquement et à titre d'exemple, deux formes d'exécution, ainsi que des variantes de l'instrument endoscopique de suture, objet de cette invention.
La figure 1 est une vue en élévation avec arrachement partiel de cet instrument illustré avec l'aiguille de suture escamotée;
la figure 2 est une vue semblable à la figure 1 où l'instrument est illustré avec l'aiguille de suture faisant saillie du corps de l'instrument;
la figure 3 est une vue en coupe selon III-III de la figure 1;
la figure 4 est une vue en coupe selon IV-IV de la figure 1;
les figures 5 et 6 sont des vues semblables aux figures 1 et 2, représentant une autre variante;
la figure 7 est une vue en coupe selon VII-VII de la figure 5;
la figure 8 est une vue en coupe selon VIII-VIII de la figure 5;
la figure 9 est une vue en élévation d'une dernière variante illustrée à l'état de flexion;
la figure 10 est une vue partielle agrandie selon la flèche X de la figure 9;
la figure 11 est une vue en élévation d'une seconde forme d'exécution de cet instrument de suture;
la figure 12 est une vue en coupe selon XII-XII de la figure 11;
les figures 13 à 15 sont des vues partielles agrandies de l'extrémité distale de l'instrument dans trois positions différentes au cours d'un processus de suture.

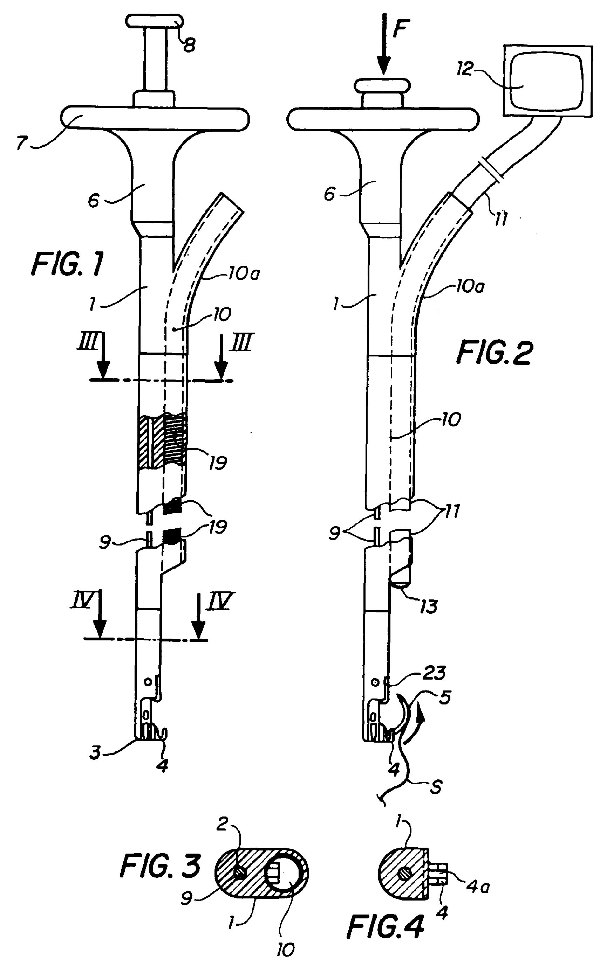

L'instrument endoscopique de suture illustré par les figures 1-4 comporte un corps creux allongé 1, présentant de préférence une certaine flexibilité, qui comporte un premier canal 2 (figure 3). L'extrémité distale 3 se termine par une pièce de guidage 4 munie d'une gorge arquée 4a (figure 4) pour le guidage d'une aiguille de suture courbe 5 (figure 2) Avantageusement il s'agit d'une aiguille de suture en deux parties de type connu, dont la pointe est amovible et sert à retenir le fil. Comme on le voit sur cette figure 2, l'aiguille de suture 5 est guidée selon une trajectoire courbe dont le rayon correspond approximativement à celui de la courbure de cette aiguille de suture 5. Le centre de cette trajectoire correspondant donc sensiblement avec celui de l'aiguille de suture 5. Cette trajectoire s'étend latéralement à l'extérieur du corps creux allongé 1, comme on le voit sur la figure 2.

L'extrémité proximale 6 du corps creux allongé 1 se termine par une tête de préhension 7. Un organe d'actionnement 8 est monté coulissant dans la tête de préhension 7. Cet organe d'actionnement 8 est relié à l'extrémité arrière de l'aiguille de suture courbe 5 par un organe de transmission souple 9, tel qu'un fil ou un câble, destiné à transmettre à l'aiguille de suture 5 les déplacements axiaux de l'organe d'actionnement 8.

Un second canal 10 est encore ménagé à travers le corps creux 1. Ce second canal 10 s'étend parallèlement au premier canal 1 sur une partie de sa longueur. Il s'arrête à une certaine distance de l'extrémité distale 3 et présente, à son autre extrémité, une partie 10a qui s'écarte du corps allongé 1. L'extrémité du canal 10 située à proximité de l'extrémité distale 3 occupe une position latérale par rapport au corps allongé 1 et à l'organe de guidage 4. Ce second canal 10 est destiné à permettre le passage d'un endoscope 11. A cet effet, la section du canal 10 est choisie pour permettre le passage d'un endoscope classique du commerce.

Dans ce cas, on peut avantageusement sélectionner un modèle d'endoscope, de la marque Pentax ou Olympus notamment, par exemple, le fibroscope d'intubation thérapeutique Olympus GIF-XQ10 de 9,8 mm de diamètre, dans lequel un canal de 2,8 mm est ménagé pour le passage d'un outil, tel qu'un outil de préhension ou de coupe.

La sortie de l'endoscope 11 peut être reliée à un appareil 12 pour former une image vidéo. A cet effet l'endoscope 11, qui ne sera pas décrit ici, comporte évidemment, de manière connue, une fibre optique terminée par une lentille 13 destinée à former une image de la zone de suture. Il est aussi possible d'observer directement l'image transmise par la fibre optique de l'endoscope par l'intermédiaire d'un simple dispositif grossissant du type binoculaire.

Pour réaliser des points de sutures, par exemple au bas de l'oesophage en vue de la fixation d'une prothèse gastrique anti-reflux, le praticien introduit le corps 1 de l'instrument à travers l'oesophage. Cette opération peut se faire sous anesthésie générale ou locale, comme on le verra par la suite. Il fait passer un endoscope dans le canal 10 et introduit le corps 1 de l'instrument à travers l'oesophage, et positionne son extrémité de manière à avoir une vision de la zone située autour du guide 4 de l'aiguille de suture courbe 5. La prothèse gastrique qui est souple peut être repliée sur elle-même et fixée à l'extrémité d'une pince (non représentée) de l'endoscope 11. Elle peut aussi, de préférence, être amenée séparément, avant l'introduction de l'instrument de suture, à travers un tube souple.

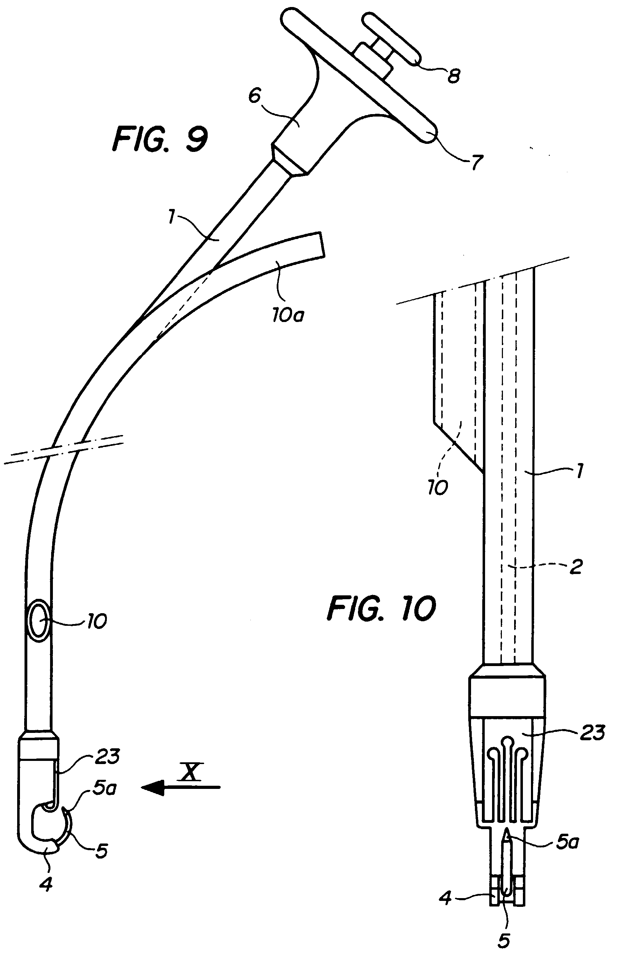

Lorsque l'extrémité distale 3 de l'instrument de suture est positionnée correctement, le praticien pousse l'organe d'actionnement 8 dans le sens de la flèche F pour amener l'aiguille courbe de suture 5 à se déplacer dans la gorge de guidage 4a afin de sortir latéralement de l'extrémité distale 3, comme illustré par la figure 2, pour pénétrer dans la paroi de la prothèse et dans le tissu contre lequel le praticien appuie cette extrémité distale 3. Une fois l'aiguille de suture courbe 5 ressortie du tissu et de la paroi de la prothèse, sa pointe amovible 5a qui retient le fil de suture S vient se fixer dans une grille 23 qui est mieux visible sur la figure 10 et tout l'instrument est alors retiré du corps du patient pour pouvoir faire un noeud sur le fil de suture. Le noeud est ensuite redescendu et serré selon une technique bien connue dans le domaine de la chirurgie endoscopique en utilisant un pousse-noeud (non représenté).

Comme on l'a dit précédemment, le corps 1 de l'instrument peut avantageusement présenter des propriétés élastiques de flexion. Dans ce cas, la force nécessaire pour faire fléchir ce corps 1 peut être choisie supérieure à celle nécessaire au percement des tissus par l'aiguille de suture 5, afin que ce corps 1 résiste à la force de réaction qui s'exerce sur lui pendant l'introduction de l'aiguille de suture 5 dans ces tissus. De préférence, les portions du corps 1 de l'instrument voisines des deux extrémités proximale 6 et distale 3 sont réalisées en un matériau rigide, par exemple en acier inoxydable.

A la place d'un corps creux 1 flexible élastiquement on peut utiliser soit un corps creux 1 souple, soit un corps creux articulé. La possibilité de déformation de ce corps est importante, du fait qu'elle facilite le passage à travers l'oesophage et évite de devoir mettre le patient sous anesthésie complète, ce qui serait nécessaire avec un corps 1 rigide, qui obligerait de basculer très fortement la tête en arrière sans pour autant permettre de former un passage rectiligne pour l'instrument.

La figure 1 montre une forme d'exécution permettant d'obtenir une flexibilité contrôlée du corps creux 1. Au moins une partie du canal 10, par exemple, traversant ce corps creux 1 et dans lequel passe l'endoscope 11 est constitué par un ressort à boudin ou une gaine souple 19. L'avantage du ressort à boudin 19 est de permettre de calculer facilement et avec précision les efforts de flexion nécessaires pour le faire fléchir élastiquement, rendant possible le choix précis des dimensions du ressort 19 en fonction des propriétés de flexion désirées.

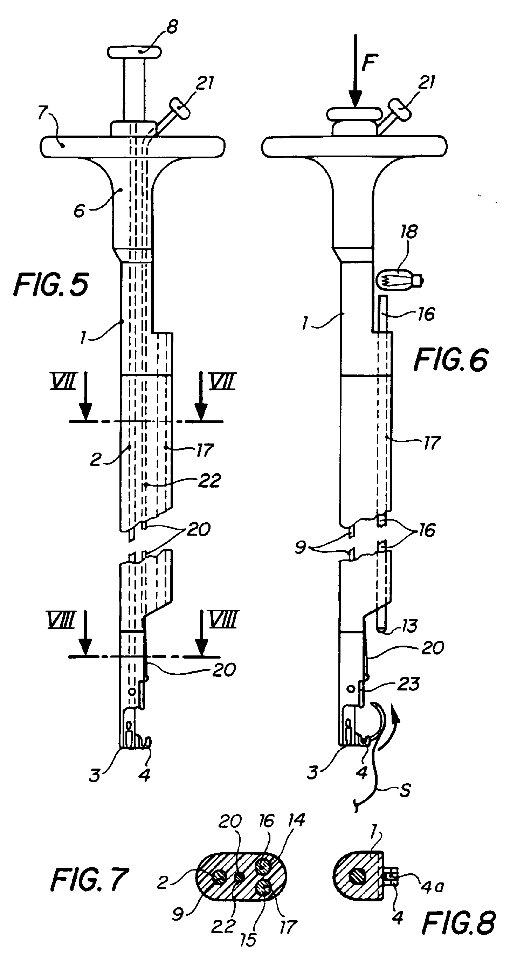

Selon une autre variante (figure 5), le corps 1 de l'instrument pourrait être articulé ou souple, des moyens (non représentés) étant prévus soit pour bloquer les articulations au moment de la suture soit comprenant un organe tracteur 21 pour permettre de faire fléchir l'extrémité distale de l'instrument par l'intermédiaire d'un fil de traction 20 fixé par exemple à la grille 23 qui sert à fixer la pointe 5a de l'aiguille de suture 5 et est désaxée par rapport à l'axe longitudinal du corps tubulaire 1. Ce fil de traction 20 peut être guidé dans un canal 22, parallèle au canal 2 et fendu longitudinalement à une certaine distance de l'extrémité distale 3, pour permettre au fil de traction 20 de s'écarter latéralement du canal 22 pour être croché à la grille 23, comme illustré par les figures 5 et 6.

Cette flexion de l'extrémité distale 3, consécutive à la traction transmise par le fil de traction 20, permet d'appliquer le guide 4 contre le tissu à suturer et de résister à la contre pression exercée sur l'extrémité distale 3 lors de l'introduction de l'aiguille de suture 5 à travers la paroi de la prothèse et le tissu adjacent de la base de l'oesophage.

Une autre variante est illustrée par les figures 5 à 7 et diffère de la forme d'exécution des figures 1-3 par le fait que le canal 10 destiné à recevoir l'endoscope 11 est remplacé par deux canaux plus petits 14 et 15 destinés au passage de deux guides de lumière 16, respectivement 17, notamment des fibres optiques, l'une 16 pour conduire la lumière d'une source de lumière 18 à l'extrémité distale du corps 1 de l'instrument pour éclairer la zone de travail de l'aiguille 5, l'autre 17 dont l'extrémité distale se termine par une lentille 13 comme dans le cas de l'endoscope 11 pour former une image de la zone de travail.

Selon une autre variante possible, le corps 1 de l'instrument pourrait être rigide et les deux canaux 16 et 17 pourraient être de nouveau remplacés par un seul canal comme dans les figures 1 à 4, mais la partie 10a du canal 10 illustrée dans ces figures 1 à 4 serait supprimée, comme illustré par les figures 5 et 6, pour ne former qu'un canal droit, destiné à recevoir un endoscope rigide. Une telle variante peut être destinée à certaines applications où il n'est pas souhaitable que le corps 1 de l'instrument soit flexible.

Selon la variante des figures 9 et 10, on voit que le canal 10 pour le passage d'un endoscope n'est plus situé dans le plan de la trajectoire de l'aiguille de suture 5, mais a été tourné de 90° autour de l'axe du corps tubulaire 1 pour se trouver placé latéralement par rapport à cette trajectoire et non plus dans son plan, ce qui permet d'avoir une meilleure vision du champ opératoire que dans les formes d'exécutions précédentes où la vision peut être gênée par la muqueuse de l'oesophage.

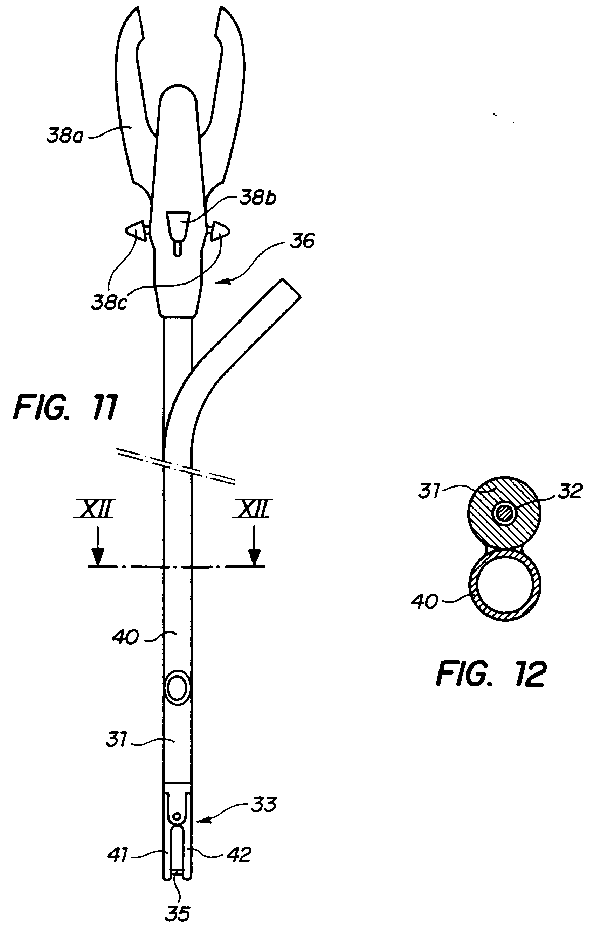

L'instrument selon la seconde forme d'exécution est dérivé d'un instrument vendu dans le commerce sous la marque Endo-Stitch^{®} de United States Surgical Corporation, Norwalk Connecticut et distribué par TYCO Healthcare Limited. Cet instrument comporte également un corps allongé 31 entre une extrémité distale 33 et une extrémité proximale 36. Cette dernière extrémité comporte des organes d'actionnement 38a, 38b, 38c pour actionner divers éléments de suture situés à l'extrémité distale 33. Ces organes de commandes 38a, 38b, 38c sont reliés à ces éléments de suture de l'extrémité distale 33, par des éléments de liaison souple (non représentés), passant le long du corps allongé 31 à l'intérieur d'un ou plusieurs canaux 32 (figure 12).

Un canal supplémentaire au moins, 40 est fixé au corps allongé 31 ou formé directement avec lui et s'étend parallèlement à ce corps allongé 31. Ce canal 40 est destiné, comme dans la forme d'exécution précédente, à permettre le passage d'un endoscope, comme dans les figures 1 à 3 ou tout au moins de deux fibres optiques, telles que les fibres optique 16 et 17 des figures 5 à 8 et remplissant le même rôle que celles-ci. Avantageusement, suivant les utilisations pour lesquelles cet instrument est prévu, le corps allongé 31 est souple. Comme dans le cas des figures 1 et 2, un ressort à boudin peut être utilisé pour conférer une élasticité à ce corps allongé souple 31.

Les éléments de suture de l'extrémité distale 33 comportent deux branches 41, 42, montées oscillantes selon un axe transversal au corps allongé 31, une aiguille de suture 35 et deux éléments de verrouillage 43, 44. Les branches 41, 42 peuvent être écartées l'une de l'autre en actionnant les organes 38a, 38b et les éléments de verrouillages 43, 44 peuvent être actionnés séparément par les deux organes d'actionnement 38c dont chacun est relié à un élément de verrouillage 43, 44.

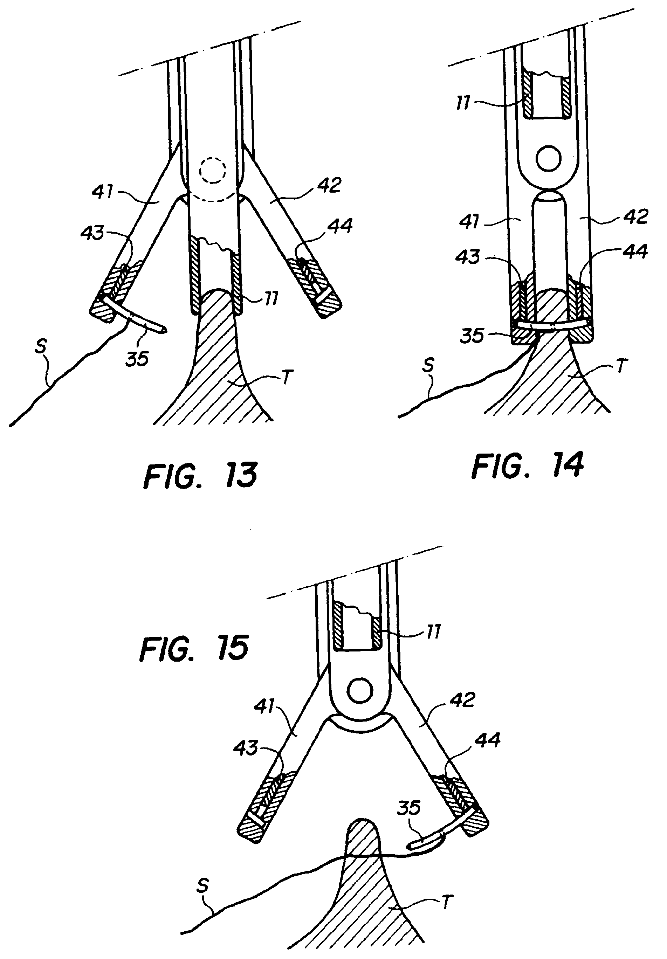

Pour effectuer une suture, on aspire la muqueuse par le canal de l'endoscope 11 comme dans le traitement des varices oesophagiennes, par ligature élastique (banding) on verrouille une des extrémités de l'aiguille de suture 35, par exemple l'extrémité gauche (figure 13) à l'aide de l'élément de verrouillage 43, l'élément de verrouillage 44 étant lui retiré pour libéré l'extrémité correspondante de l'aiguille de suture 35. Ensuite on serre les branches 41, 42 l'une contre l'autre et faisant passer l'aiguille de suture 35 à travers le tissu à suturer T (figure 14). On pousse alors l'élément de verrouillage 44 de l'aiguille de suture 35 en avant et on retire l'élément de verrouillage 43 pour libérer l'autre extrémité de cette aiguille de suture 35, de sorte qu'en écartant à nouveau les branches 41, 42 l'une de l'autre, l'aiguille de suture 35 ressort de l'autre côté du tissu à suturer T (figure 15). Le fil de suture S a donc traversé ce tissu T. L'instrument peut alors être ressorti du corps du patient pour faire un noeud.

Bien que la description qui précède parle essentiellement, à titre d'exemple, de la fixation d'une prothèse anti-reflux à la base de l'oesophage, il est évident que l'invention n'est nullement limitée à cette application particulière, même si l'utilisation de cet instrument présente des avantages évidents pour cette application. Cet instrument est utilisable chaque fois qu'il est nécessaire de pratiquer une suture d'une plaie « naturelle » comme un ulcère ou chirurgicale après excision d'une lésion ainsi que la pose d'un capteur, notamment en ne passant qu'à travers un canal naturel du corps humain ou de celui d'un animal. Un tel instrument peut encore trouver une utilisation pour des interventions relatives à l'obésité, où les deux parois du haut de l'estomac sont suturées pour réduire la capacité de rétention gastrique. L'utilisation de cet instrument en laparoscopie est également envisageable avec profit dans la mesure où il permet de ne pratiquer qu'une incision et non deux comme ceci est nécessaire avec les instruments de l'art antérieur.

## Revendications

1. Instrument endoscopique de suture, comprenant un corps creux allongé (1, 31) dont une extrémité distale (3, 33) comporte une aiguille de suture (5, 35) et dont l'extrémité proximale (6) comporte des moyens d'actionnement (8, 38) pour commander ladite aiguille de suture (5, 35) des moyens de transmission (9) reliant ledit organe d'actionnement (8) à ladite extrémité distale (3, 33) à travers un canal longitudinal (2, 32) traversant ledit corps creux (1), caractérisé en ce que ledit corps creux (1, 31) comporte au moins un second canal longitudinal (10, 40) dont une première extrémité s'arrête à proximité de ladite extrémité distale (3, 33), occupe une position latérale à l'axe longitudinal dudit corps creux (1) et est dimensionné pour permettre le passage d'au moins deux guides d'ondes lumineuses (11, 16, 17), l'un (11, 16) pour conduire la lumière d'une source lumineuse (18) de l'extrémité proximale (6, 36) à l'extrémité distale (3, 33) et l'autre (11, 17) comportant une lentille (13) à son extrémité distale pour former une image de la zone éclairée par la lumière issue de l'extrémité distale dudit premier guide d'ondes optiques (11, 16).

2. Instrument selon la revendication 1, caractérisé en ce que ladite extrémité distale (3) dudit corps creux allongé (1) comporte une aiguille de suture courbe (5) et des moyens de guidage (4) pour guider cette aiguille de suture (5) selon une trajectoire centrée sensiblement au centre de sa courbure, s'étendant latéralement hors dudit corps creux allongé (1) et dont l'extrémité proximale (6) comporte un organe d'actionnement (8) pour déplacer ladite aiguille de suture (5) par rapport auxdits moyens de guidage (4) .

3. Instrument selon l'une des revendications précédentes, caractérisé en ce que au moins une portion dudit corps creux allongé (1) est flexible.

4. Instrument selon l'une des revendications précédentes, caractérisé en ce qu'au moins un des canaux (10) traversant ledit corps creux (1) est formé au moins partiellement par un ressort à boudin (11).

5. Instrument selon la revendication 4, caractérisé en ce que ledit ressort à boudin (19) est noyé dans un corps en matière plastique souple dans lequel est ménagé ledit second canal (10).

6. Instrument selon l'une des revendications précédentes, caractérisé en ce que ledit corps creux (1) présente des propriétés élastiques de flexion susceptibles de permettre la flexion dudit corps creux (1) au-delà d'un seuil supérieur à la force de réaction appliquée transversalement au dit corps creux (1) consécutivement à la pénétration de ladite aiguille de suture (5) dans les tissus à suturer.

7. Instrument selon l'une des revendications précédentes, caractérisé en ce que la section dudit second canal (10) est dimensionné pour recevoir un endoscope (11).

8. Instrument selon l'une des revendications 1 à 6, caractérisé en ce qu'il comporte un second (14) et un troisième (15) canal pour recevoir respectivement lesdits deux guides d'ondes (16, 17).

9. Instrument selon l'une des revendications 1, 2, 6, 7, caractérisé en ce que ledit corps creux (1) est souple et comporte des moyens de traction (20) dont une extrémité est solidaire d'un organe de traction (21) et dont l'autre extrémité est fixée à ladite extrémité distale (3), en un point désaxé par rapport à l'axe longitudinal dudit corps creux (1) et situé du côté de ladite trajectoire de l'aiguille de suture (5).

10. Instrument selon l'une des revendications 1-5, 7-9, caractérisé en ce que ladite aiguille de suture (35) est montée entre deux branches oscillantes (41, 42) susceptibles de s'écarter et de se rapprocher l'une de l'autre, lesdits moyens d'actionnement (38) étant reliés à des éléments de verrouillage (43, 44) de ladite aiguille de suture (35) dans chacune desdites branches (41, 42).
